# FASCICULE DE BREVET EUROPEEN

(11) **EP 1 185 323 B1**
(45) Date de publication et mention de la délivrance du brevet: **12.02.2003**
(21) Numéro de dépôt: 00931348.7
(22) Date de dépôt: 25.05.2000
(51) Int. Cl.: A61M 5/32

(54) **DISPOSITIF D'INJECTION A USAGE UNIQUE**
EINWEGINJEKTIONSVORRICHTUNG
DISPOSABLE INJECTION DEVICE

(30) Priorité: 10.06.1999 FR 9907334
(43) Date de publication de la demande: 13.03.2002
(73) Titulaire: SANOFI-SYNTHELABO, 75035 Paris (FR)
(72) Inventeur: BRUNEL, Marc, F-31000 Toulouse (FR)
(74) Mandataire: Cabinet BARRE LAFORGUE & associés
(86) Numéro de dépôt international: FR0001420
(87) Numéro de publication internationale: WO00076565

(56) Documents cités:
- EP-A- 0 966 983
- WO-A-93/00949
- WO-A-99/37345
- FR-A- 2 741 268
- US-A- 4 863 434
- US-A- 5 188 614

## Description

L'invention concerne un dispositif d'injection à usage unique conçu pour être pré-rempli d'une dose de liquide à injecter.

Les dispositifs d'injection à usage unique utilisés traditionnellement à l'heure actuelle consistent en des seringues pré-remplies dotées d'un capuchon destiné à protéger l'aiguille avant utilisation, puis retiré en vue de l'injection, et enfin remis en place après injection en vue d'éviter les risques ultérieurs de piqûre avec l'aiguille souillée.

Il s'est toutefois avéré qu'une telle conception présente un inconvénient majeur résultant du fait que, lors du recapuchonnage de l'aiguille, le capuchon doit être présenté en regard de l'extrémité de cette aiguille. Cette obligation s'est en effet avérée être la cause d'accidents par piqûre relativement fréquents avec tous les risques que comportent de telles piqûres avec une aiguille souillée. De plus, de telles seringues ne présentent aucune sécurité visant à interdire leur réutilisation et doivent donc être l'objet de soins particuliers en vue de leur destruction.

En vue de pallier ces inconvénients, la technique habituelle consiste à doter les dispositifs d'injection d'un étui protecteur apte à coulisser le long du corps de seringue ou à l'intérieur duquel la seringue peut se rétracter, de façon à obtenir soit une position d'injection où l'aiguille d'injection est dégagée, soit une position de protection après usage où cette aiguille d'injection est logée et protégée à l'intérieur de l'étui protecteur.

Selon cette conception en outre, quatre principes généraux différents sont proposés à l'heure actuelle pour engendrer le coulissement de l'étui protecteur ou de la seringue après usage.

Selon une première solution, des moyens de blocage relatif en translation sont ménagés sur le corps de seringue et à l'intérieur de l'étui protecteur, de façon à permettre d'amener manuellement ledit étui à coulisser entre ses positions d'injection et de protection après usage. Selon cette solution, ce coulissement est obtenu en maintenant le corps de seringue d'une main et en déplaçant au moyen de l'autre main, l'étui protecteur en direction de l'aiguille d'injection.

Cette première solution conduit à la réalisation de dispositifs d'injection de conception simple tels que ceux décrits dans le brevet FR 2.733.687, entraînant un surcoût de production à une échelle industrialisée relativement peu important par rapport au coût de production d'une seringue pré-remplie classique dépourvue de protection. Par contre, de tels dispositifs d'injection n'offrent pas une sécurité absolue pour leur utilisateur vis-à-vis des risques de piqûres. En effet, en vue de faire coulisser l'étui protecteur, après injection, l'utilisateur est tenu d'opérer une traction manuelle sur cet étui, et donc d'apposer les doigts sur ce dernier, à une distance relativement faible de l'aiguille d'injection. De ce fait, et plus particulièrement dans le cas de dispositifs d'injection de faibles dimensions destinés à l'injection de petites doses de liquides, tout glissement des doigts sur l'étui protecteur peut conduire l'utilisateur à se piquer avec l'aiguille d'injection.

Selon une deuxième solution, le dispositif d'injection comporte des moyens élastiques interposés entre le corps de seringue et l'étui protecteur, agencés pour être maintenus comprimés et assurer un positionnement de l'étui protecteur et de la seringue dans leur position d'injection, avant et en cours d'injection, et pour se détendre et engendrer automatiquement le coulissement dudit étui protecteur ou de ladite seringue vers leur position de protection après usage, en fin d'injection.

Cette deuxième solution a quant à elle permis de réaliser des dispositifs d'injection tels que décrits dans les demandes de brevet internationales WO 91.13643, WO 93.23098, WO 93.25254 et européennes EP 446.511 et EP 307.367, garantissant contre tout risque de piqûre accidentelle avant et après injection, du fait notamment que l'étui protecteur ou la seringue sont amenés à coulisser de façon automatique grâce au déclenchement des moyens élastiques, après injection.

De tels dispositifs d'injection présentent toutefois un inconvénient majeur provenant du fait que lors du déclenchement des moyens élastiques, commandé par des moyens de déverrouillage disposés sur la tige de piston, il n'est aucunement garanti que la seringue a été entièrement vidée du fait des tolérances admises concernant la longueur des seringues lors de la fabrication de ces dernières. Or, de tels dispositifs d'injection étant notamment destinés à être utilisés en vue de l'injection de très faibles doses de liquides (de l'ordre du millimètre cube) dont la quantité administrée doit être rigoureusement respectée, cette solution technique s'avère inapte à satisfaire les exigences requises.

Une troisième solution, notamment décrite dans le brevet US 3.046.985, a consisté à doter le dispositif d'injection de moyens de déclenchement indépendants actionnables par une action séparée de celle engendrant l'injection du liquide. Il s'est toutefois avéré que de tels dispositifs d'injection, qui nécessitent une action spécifique s'écartant du geste naturel utilisé pour l'injection, ont été mal accueillis par le personnel médical et les malades s'auto-administrant leur médicament, et sont en fait très peu utilisés.

La quatrième solution, décrite notamment dans les brevets US 4.767.413 et WO 92/17229 a enfin consisté à doter le dispositif d'injection d'organes de blocage manuel aptes à permettre de maintenir par pression des doigts l'étui protecteur dans sa position d'injection, et à autoriser son déplacement vers sa position de protection après usage par relâchement de la pression manuelle exercée. Cette solution s'est toutefois également heurtée à un accueil négatif du personnel médical et des malades et s'avère peu prisée à l'heure actuelle.

WO-A-93 00 949 divulgue un dispositif d'injection conforme au préambule de la revendication 1.

La présente invention vise à pallier l'ensemble des inconvénients précités des techniques de protection après usage des dispositifs d'injection, et a pour principal objectif de fournir un dispositif d'injection conduisant à administrer le liquide selon un geste naturel et garantissant l'injection totale de la dose de liquide.

Un autre objectif de l'invention est de fournir un dispositif d'injection non réutilisable après usage.

A cet effet, l'invention vise un dispositif d'injection à usage unique comprenant :
- une seringue comportant une aiguille d'injection, et délimitant une chambre remplie d'une dose de liquide à injecter, obturée par un piston solidaire d'une des extrémités d'une tige de piston dotée d'un poussoir au niveau de son extrémité opposée,
- un corps de seringue solidaire de la seringue,
- un étui de protection,
- des moyens de blocage relatif en translation ménagés sur le corps de seringue et l'étui de protection, pour permettre un déplacement relatif dudit étui de protection et de l'ensemble seringue/corps de seringue entre une position d'injection où l'aiguille d'injection est dégagée et une position de protection après usage où ladite aiguille d'injection est logée à l'intérieur de l'étui de protection,
- des moyens élastiques disposés entre le corps de seringue et l'étui de protection de façon à être comprimés dans la position d'injection de ces derniers.

Selon l'invention, ce dispositif d'injection se caractérise en ce qu'il comprend :
- une bague de verrouillage dotée de moyens d'appui digital pour l'injection liquide, et d'organes de blocage en translation de ladite bague aptes à la maintenir dans une position où elle verrouille les moyens de blocage en translation de l'étui de protection et de l'ensemble seringue/corps de seringue, dans la position d'injection de ces derniers, en l'absence d'effort exercé sur les moyens d'appui digital et pour un effort exercé sur ces derniers équivalent à la force de réaction engendrée conduisant au coulissement du piston, et pour autoriser un déplacement axial de ladite bague vers une position où elle libère lesdits moyens de blocage et autorise le déplacement relatif de l'étui de protection et de l'ensemble seringue/corps de seringue vers la position de protection après usage de ces derniers, grâce à l'action des moyens élastiques, pour un effort exercé sur les moyens d'appui digital supérieur à la force de réaction engendrée en vue du coulissement du piston, et obtenue une fois ledit piston parvenu en fin de course.

Le dispositif d'injection selon l'invention présente donc l'originalité de comporter une bague de verrouillage mobile portant les moyens d'appui digital et adaptée pour :
- maintenir verrouillés dans leur position d'injection l'étui de protection et l'ensemble seringue/corps de seringue, avant injection et en cours d'injection, du fait que la force exercée sur le poussoir de la tige de piston et la force de réaction appliquée sur les moyens d'appui digital, s'avère Uniquement entraîner le coulissement du piston à l'intérieur de la seringue et donc l'injection du liquide,
- se déplacer axialement et donc libérer les moyens de blocage en translation de l'étui de protection et de l'ensemble seringue/corps de seringue, uniquement lorsque le piston est parvenu en fin de course, c'est-à-dire lorsque ledit piston étant en butée dans la seringue, la force de réaction s'applique entièrement sur les moyens d'appui digital et s'avère alors suffisante pour déplacer la bague de verrouillage.

Ainsi, le déclenchement de la protection est obtenu par le biais du geste naturel conduisant à l'injection du liquide, et ce uniquement une fois le piston parvenu en fin de course, garantissant ainsi l'administration de l'intégralité de la dose de liquide.

Selon un premier mode de réalisation avantageux, les moyens élastiques sont disposés entre le corps de seringue et l'étui de protection de façon à engendrer un retrait de l'ensemble seringue/corps de seringue à l'intérieur dudit étui de protection après déplacement axial de la bague de verrouillage.

Selon ce mode de réalisation, l'ensemble seringue/corps de seringue vient se rétracter à l'intérieur de l'étui de protection, d'une part après déplacement axial de la bague de verrouillage, et d'autre part lorsque l'utilisateur cesse d'exercer une pression sur le poussoir de la tige de piston. De ce fait, le déclenchement s'opère en douceur sans heurt pour le patient.

Selon ce mode de réalisation, en outre, le corps de seringue comporte avantageusement une bague tubulaire logée à l'intérieur de l'étui de protection, lesdits bague et étui de protection comprenant des épaulements internes entre lesquels s'étendent les moyens élastiques. De plus, la bague de verrouillage est alors montée autour de l'étui de protection de façon à pouvoir coulisser axialement par rapport à ce dernier.

Le mécanisme de déclenchement se trouve ainsi masqué à la vue de l'utilisateur et n'est donc pas susceptible de provoquer un blocage chez un malade devant s'auto-administrer une dose de médicament.

De plus, et de façon avantageuse, les moyens de blocage relatif en translation de la bague tubulaire et de l'étui de protection sont ménagés de façon que, dans la position de protection après usage, ladite bague tubulaire s'étende au moins partiellement dans le prolongement postérieur dudit étui de protection.

Dans ce cas, la bague annulaire présente en outre avantageusement au moins une surface externe de couleur différente de celle de la bague de verrouillage et de l'étui de protection. De ce fait, l'identification d'un dispositif d'injection utilisé est immédiate.

Par ailleurs et de façon avantageuse, l'étui de protection comporte au moins une languette déformable radialement portant un élément de blocage relatif en translation dudit étui de protection dans sa position d'injection, la bague de verrouillage étant adaptée pour enserrer chacune desdites languettes dans sa position de verrouillage, et pour libérer chaque languette de façon à autoriser son expansion radiale, lors de son déplacement axial.

De plus et de façon avantageuse, les moyens de blocage relatif en translation de la bague tubulaire et de l'étui de protection, dans la position d'injection de ces derniers, comportent, pour chaque languette, un bossage interne ménagé sur cette dernière et une nervure externe ménagée sur la bague tubulaire, lesdits bossage et nervure présentant des portées d'appui inclinées conjuguées.

De même, les moyens de blocage relatif en translation de la bague tubulaire et de l'étui de protection, dans la position de protection après usage de ces derniers, comprennent avantageusement une nervure interne ménagée vers l'extrémité postérieure dudit étui de protection, présentant une portée d'appui inclinée conjuguée de celle de la nervure externe précitée de la bague tubulaire.

Par ailleurs, l'étui de protection comporte avantageusement au moins une languette déformable radialement, ménagée à une distance de la nervure postérieure dudit étui de protection adaptée pour assurer le blocage en translation de la nervure externe de la bague tubulaire, chacune desdites languettes présentant une position naturelle expansée radialement apte à permettre le montage initial de la bague tubulaire, et étant adaptée pour être rétractée radialement de façon à venir en saillie à l'intérieur de l'étui de protection, lors du montage de la bague de verrouillage.

De telles languettes sont conçues d'une part pour autoriser le montage de la bague tubulaire grâce à leur réalisation en position radialement expansée, puis pour venir faire saillie à l'intérieur de l'étui de protection, après mise en place de la bague de verrouillage, de façon à constituer des éléments anti-retour empêchant de réutiliser le dispositif d'injection.

De façon avantageuse, la bague de verrouillage comporte également un tronçon tubulaire postérieur agencé pour ceinturer la nervure interne postérieure de l'étui de protection, de façon à fretter cette dernière, et à éviter tout risque d'échappement de la bague tubulaire.

Cette bague de verrouillage comporte également, de façon avantageuse, un épaulement interne ménagé au niveau de son tronçon tubulaire postérieur, l'étui de protection comportant un bossage de butée dudit épaulement après déplacement axial de ladite bague de verrouillage.

L'étui de protection comporte, quant à lui avantageusement, un tronçon postérieur de diamètre adapté pour loger la bague tubulaire et le long duquel s'étend la bague de verrouillage, et un tronçon antérieur de diamètre conjugué du diamètre externe de la seringue, lesdits tronçons postérieur et antérieur étant séparés par un épaulement interne d'appui des moyens élastiques.

Afin d'assurer la solidarisation de la bague annulaire sur la seringue, cette seringue comporte avantageusement une collerette postérieure, la bague tubulaire comportant une gorge annulaire interne postérieure de section adaptée pour loger ladite collerette.

L'étui de protection comporte, par ailleurs, avantageusement une nervure annulaire externe, les moyens de verrouillage de la bague de verrouillage consistant en une gorge annulaire interne ménagée dans ladite bague de verrouillage, et de section adaptée pour loger ladite nervure externe.

Selon un deuxième mode de réalisation avantageux, les moyens élastiques sont disposés entre le corps de seringue et l'étui de protection de façon à engendrer le coulissement dudit étui de protection le long de la seringue, après déplacement axial de la bague de verrouillage.

Le déclenchement obtenu, en continuant à exercer une pression sur le poussoir de la tige de piston une fois le piston parvenu en fin de course, conduit dans ce cas à amener l'étui de protection à coulisser le long de l'ensemble seringue/corps de seringue jusqu'à une position où l'aiguille de protection se trouve logée dans ledit étui de protection.

Selon ce mode de réalisation, en outre, le corps de seringue comporte avantageusement un fourreau tubulaire à l'intérieur duquel est logé l'étui de protection, les moyens élastiques s'étendant dans ledit fourreau tubulaire dans le prolongement postérieur dudit étui de protection. De plus, la bague de verrouillage est alors montée autour du fourreau tubulaire de façon à pouvoir coulisser axialement par rapport à ce dernier.

Par ailleurs, et de façon avantageuse, le fourreau tubulaire comporte au moins une languette déformable radialement portant un élément de blocage relatif en translation dudit fourreau dans sa position d'injection, la bague de verrouillage étant adaptée pour enserrer chacune desdites languettes dans sa position de verrouillage, et pour libérer chaque languette de façon à autoriser son expansion radiale, lors de son déplacement axial.

De plus, de façon avantageuse, les moyens de blocage relatif en translation du fourreau tubulaire et de l'étui de protection, dans la position d'injection de ces derniers, comportent, pour chaque languette, un bossage interne ménagé sur cette dernière et une nervure externe ménagée sur l'étui de protection, lesdits bossage et nervure présentant des portées d'appui inclinées conjuguées.

En outre, les moyens de blocage relatif en translation du fourreau tubulaire et de l'étui de protection, dans la position de protection après usage de ces derniers, comprennent avantageusement une seconde nervure externe ménagée sur l'étui de protection et délimitant une gorge avec la première nervure externe, et une nervure interne ménagée au niveau de l'extrémité antérieure du fourreau tubulaire, de forme adaptée pour venir se loger dans la gorge.

Le fourreau tubulaire comporte, quant à lui de façon avantageuse, une nervure externe ménagée sur ledit fourreau au niveau de chaque languette, les moyens de verrouillage de la bague de verrouillage consistant en une gorge annulaire interne délimitée par une nervure antérieure agencée pour se trouver en butée sous la nervure du fourreau tubulaire.

Ce fourreau tubulaire comprend également avantageusement une nervure annulaire externe postérieure de butée de la bague de verrouillage après déplacement axial de cette dernière.

Enfin, afin d'assurer la solidarisation du fourreau tubulaire sur la seringue, cette seringue comporte avantageusement une collerette postérieure, le fourreau tubulaire comportant une gorge annulaire interne postérieure de section adaptée pour loger ladite collerette.

D'autres caractéristiques, buts et avantages de l'invention ressortiront de la description détaillée qui suit en référence aux dessins annexés qui en représentent à titres d'exemples non limitatifs deux modes de réalisation préférentiels. Sur ces dessins :
- la figure 1 est une coupe longitudinale par un plan axial d'un premier mode de réalisation d'un dispositif d'injection conforme à l'invention, dans la position d'injection de ce dernier,
- la figure 2 en est une coupe longitudinale par un plan axial, dans la position de protection après usage de ce dispositif d'injection,
- la figure 3 est une coupe longitudinale par un plan axial B de l'étui de protection de ce dispositif d'injection,
- la figure 4 est une coupe longitudinale par un plan axial A de cet étui de protection,
- la figure 5 est une coupe longitudinale par un plan axial de la bague tubulaire de ce dispositif d'injection,
- la figure 6 est une coupe longitudinale par un plan axial de la bague de verrouillage de ce dispositif d'injection,
- la figure 7 est une coupe longitudinale par un plan axial d'un deuxième mode de réalisation d'un dispositif d'injection conforme à l'invention, dans la position d'injection de ce dernier,
- et la figure 8 est une coupe longitudinale par un plan axial de ce deuxième mode de réalisation, dans la position de protection après usage du dispositif d'injection.

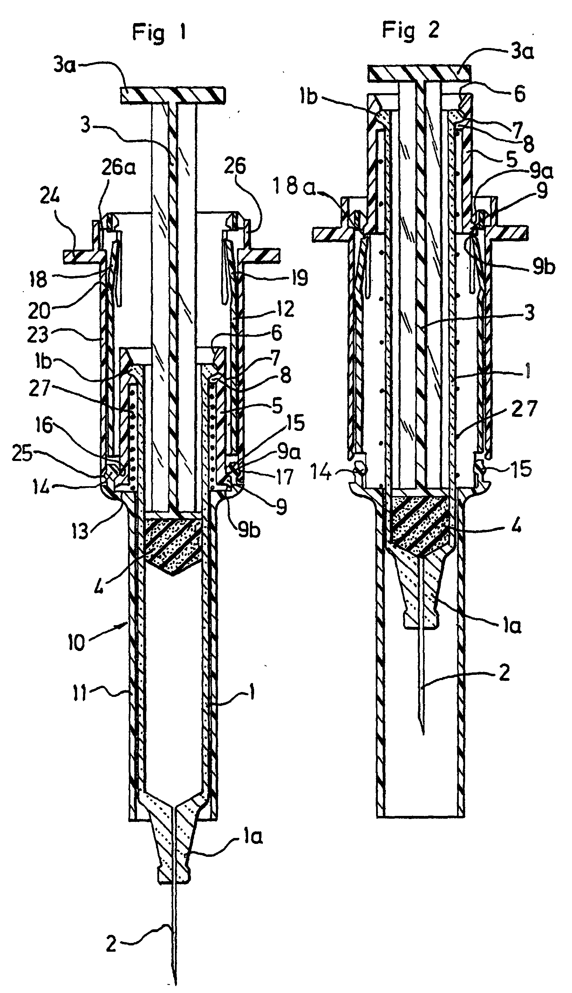

Les dispositifs d'injection conformes à l'invention représentés respectivement aux figures 1, 2 et 7, 8 comprennent tous deux, en premier lieu, une seringue pré-remplie 1 de type traditionnel, telle que par exemple réalisée en verre, comportant de façon classique un nez antérieur 1a sur lequel est montée une aiguille 2, et une collerette 1b au niveau de son extrémité postérieure.

Cette seringue 1 comporte également de façon classique, une tige de piston 3 dotée d'un poussoir 3a au niveau de son extrémité postérieure, et actionnant un piston 4 solidarisé à son extrémité antérieure.

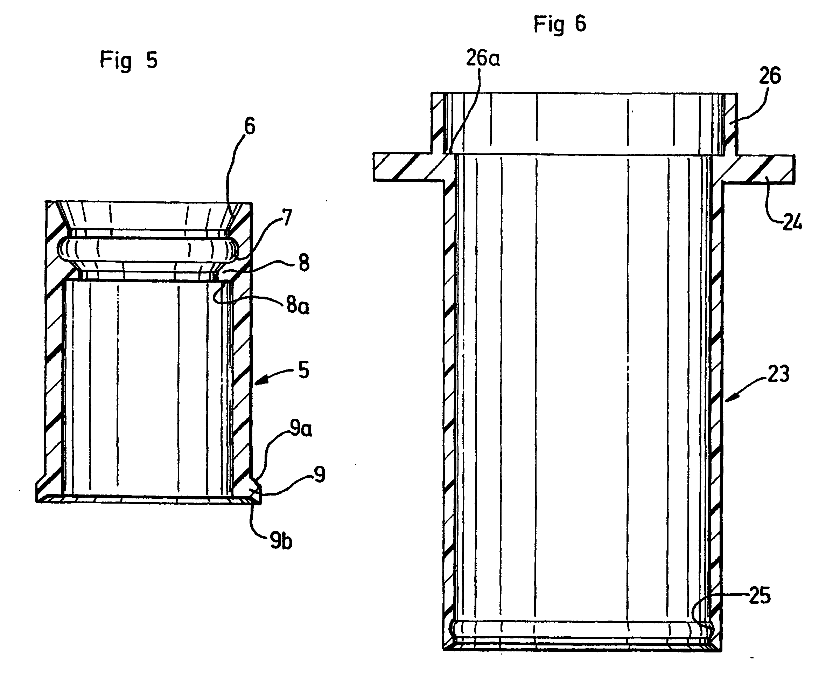

Le dispositif d'injection représenté aux figures 1 et 2 comprend, en outre, un corps de seringue destiné à être solidarisé à la seringue 1, constitué d'une bague tubulaire 5 représentée en détail à la figure 5.

Cette bague tubulaire 5 comporte, en premier lieu, une portion d'extrémité postérieure 6 de forme tronconique formant une rampe permettant de l'amener à franchir la collerette 1b de la seringue 1, et juxtaposée à ladite portion tronconique, une gorge 7 de section adaptée pour loger la dite collerette, délimitée par une nervure annulaire interne 8 présentant, à l'opposé de ladite gorge, une portée d'appui 8a radiale.

Cette bague tubulaire 5 comporte, en outre, au niveau de son extrémité antérieure, une nervure annulaire externe 9 présentant deux faces frontales 9a, 9b parallèles et inclinées par rapport à l'axe de ladite bague tubulaire.

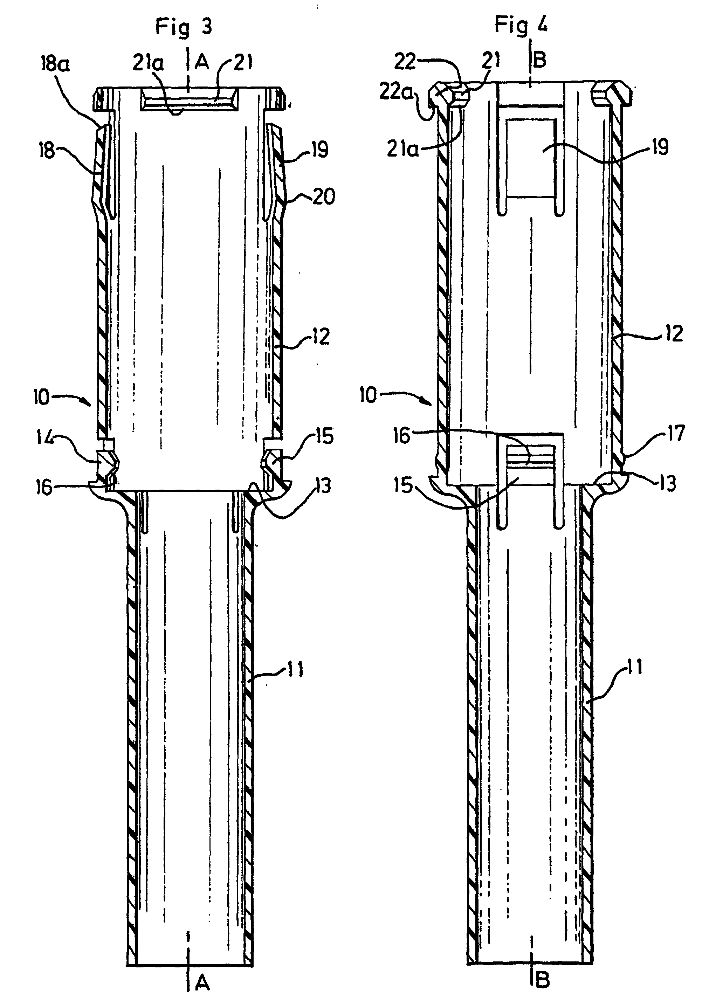

Le dispositif d'injection comprend, en outre, un étui de protection 10 représenté aux figures 3 et 4, adapté pour loger la bague tubulaire 5, et à l'intérieur duquel vient se rétracter la seringue 1 après utilisation.

Cet étui de protection 10 est composé de deux tronçons longitudinaux 11, 12 séparés par un épaulement interne 13 radial :
- un tronçon antérieur 11 de diamètre interne conjugué du diamètre externe de la seringue 1,
- un tronçon postérieur 12 de diamètre interne sensiblement supérieur au diamètre externe de la bague tubulaire 5, adapté pour loger cette dernière.

L'étui de protection 10 comprend, en premier lieu, deux languettes 14, 15 déformables radialement disposées de façon diamétralement opposée au voisinage de l'extrémité antérieure du tronçon postérieur 12, et délimitées chacune par une découpe en forme de U inversé ménagée dans la paroi périphérique dudit étui de protection.

Chacune de ces languettes 14, 15 présente une face interne en forme générale de dièdre formant une face frontale antérieure 16 inclinée, adaptée pour constituer une portée d'appui pour la face frontale postérieure 9b de la nervure 9, permettant de bloquer en translation la bague tubulaire 5.

Cet étui de protection 10 comporte, en outre, un bossage externe annulaire 17 ménagé sensiblement à mi-hauteur des languettes 14, 15 et interrompu au niveau de ces dernières.

Cet étui de protection 10 comporte, par ailleurs, deux autres languettes 18, 19 déformables radialement, disposées dans le tiers postérieur du tronçon postérieur 12 à faible distance de l'extrémité de ce dernier, chacune coaxialement avec une des languettes 14, 15, et délimitées par des découpes en forme de U inversé ménagées dans la paroi périphérique dudit étui de protection.

Chacune de ces languettes 18, 19, dites languettes anti-retour, est réalisée lors du moulage, en position "ouverte", c'est-à-dire de façon à ne pas faire saillie à l'intérieur du tronçon postérieur 12 et à permettre ainsi le montage de la bague tubulaire 5 à l'intérieur dudit tronçon postérieur.

Afin de pouvoir être rétractée de façon que la tranche d'extrémité 18a de ces languettes 18, 19 vienne former une butée anti-retour à l'intérieur du tronçon postérieur 12, tel qu'explicité plus loin, chacune de ces languettes 18, 19 présente, en outre, un bossage externe 20 permettant de les rabattre lors du montage de la bague de verrouillage décrite ci-après.

L'étui de protection 10 comporte par ailleurs, au niveau de l'extrémité postérieure du tronçon postérieur 12, et décalés de 90 degrés par rapport aux languettes anti-retour 18, 19 :
- deux bossages internes 21 possédant chacun une face frontale antérieure 21a inclinée, adaptée pour constituer une portée d'appui pour la face frontale postérieure 9b de la nervure 9 de la bague tabulaire 5,
- deux bossages externes 22 possédant chacun une face frontale antérieure 22a de butée radiale.

Le dispositif d'injection comprend, enfin, une bague de verrouillage 23 de diamètre interne et de longueur conjugués du diamètre externe et de la longueur du tronçon postérieur 12 de l'étui de protection 10, adaptée en premier lieu pour rétracter radialement les languettes anti-retour 18, 19 lors de son montage le long dudit tronçon postérieur.

Cette bague de verrouillage 23 comprend, en premier lieu, au niveau de son extrémité postérieure, une collerette externe 24 d'appui digital. Elle comprend, en outre, au niveau de son extrémité antérieure, une gorge annulaire interne 25 de section adaptée pour loger le bossage 17 de l'étui de protection 10.

Cette bague de verrouillage 23 comprend, enfin, un tronçon cylindrique 26 s'étendant dans le prolongement postérieur de la collerette d'appui digital 24, et délimitant, en outre avec cette dernière, un épaulement interne 26a de butée radiale.

La fabrication du dispositif d'injection décrit ci-dessus comprend les étapes principales suivantes.

En premier lieu, un ressort 27 est logé dans la bague tubulaire 5, et cette dernière est mise en place dans le tronçon postérieur 12 de l'étui de protection 10, de façon à amener la face postérieure 9a de la nervure 9 à se bloquer sous la face antérieure 16 des languettes 14, 15. Dans cette position, le ressort 27 se trouve comprimé entre l'épaulement 13 de l'étui de protection 10 et la portée d'appui radiale 8a de la nervure interne 8 de la bague tubulaire 5.

Il est à noter, en outre, tel que précité, que l'introduction de la bague tubulaire 5 à l'intérieur de l'étui de protection 10 est autorisée grâce à la position "ouverte" obtenue lors du moulage des languettes anti-retour 18, 19.

La bague de verrouillage 23 est ensuite montée le long du tronçon postérieur 12 de l'étui de protection 10, jusqu'à amener la gorge 25 à loger le bossage externe 17 dudit étui de protection, position dans laquelle ladite bague de verrouillage verrouille le blocage en translation de la bague tubulaire 5 par rapport à l'étui de protection 10, obtenu par portée de la face antérieure 16 des languettes 14, 15 sur la face postérieure 9a de la nervure 9. Il est à noter, en outre, que lors de la mise en place de la bague de verrouillage 23, cette dernière contraint les languettes anti-retour 18, 19 à se rétracter radialement par contact avec le bossage 20 desdites languettes.

La dernière opération consiste à intégrer la seringue pré-remplie 1, en introduisant cette dernière dans le tronçon postérieur 12 de l'étui de protection 10 jusqu'à amener la collerette 1b à se bloquer dans la gorge interne 7 de la bague tubulaire 5, position dans laquelle l'aiguille d'injection 2 s'étend dans le prolongement antérieur dudit étui de protection. Il est à noter que lors de la mise en place de cette seringue 1, la présence de la rampe tronconique 6 facilite l'introduction de cette dernière à l'intérieur de la bague tubulaire 5.

Le dispositif d'injection obtenu, représenté à la figure 1, est ensuite utilisé de façon classique en vue d'une injection, en exerçant une force d'appui sur le poussoir 3a de la tige de piston et une force de réaction d'appui sur la collerette d'appui digital 24 de la bague de verrouillage 23.

Lors de cette injection, la résistance s'opposant au déplacement du piston 4 étant naturellement inférieure à la force à exercer pour déplacer axialement la bague de verrouillage 23, cette dernière reste positionnée dans sa position de verrouillage des organes 9, 14, 15 de blocage relatif en translation de la bague tubulaire 5 et de l'étui de protection 10.

Par contre, une fois le piston 4 parvenu en fin de course, c'est-à-dire une fois l'intégralité de la dose de liquide injectée, les mêmes forces d'appui et de réaction conduisent à faire remonter axialement la bague de verrouillage 23 le long du tronçon postérieur 12 de l'étui de protection 10, jusqu'à amener l'épaulement 26a de cette dernière en butée radiale contre les bossages externes 22 dudit étui de protection.

Les languettes 14, 15 de l'étui de protection 10 se trouvent alors déverrouillées, et lorsque l'utilisateur relâche la pression exercée sur le poussoir 3a de la tige de piston 3, l'ensemble bague tubulaire 5/seringue 1 est amené à se rétracter à l'intérieur de l'étui de protection 10 sous l'action du ressort 27.

Tel que représenté à la figure 2, et en fin de rétraction, la face postérieure 9a de la nervure 9 de la bague tubulaire 5 vient en butée contre la face antérieure 21a des bossages internes 21 de l'étui de protection 10, position dans laquelle ladite nervure 9 se trouve bloquée en translation dans les deux sens entre les bossages 21 et les languettes anti-retour 18, 19 dudit étui de protection.

Il est à noter, en outre, que le tronçon cylindrique 26 de la bague de verrouillage 23 forme une frette autour de la portion postérieure du tronçon postérieur 12 de l'étui de protection 10 dans laquelle sont ménagées les bossages 21, et garantit donc contre tout risque d'échappement de la bague tubulaire 5, en fin de course de cette dernière.

Il est également à noter que, la bague tubulaire 5 s'étendant dans le prolongement postérieur de l'étui de protection, 10, après utilisation, cette dernière peut avantageusement présenter une couleur différente de celle des autres éléments, de façon à permettre l'identification immédiate des dispositifs d'injection utilisés.

Le dispositif d'injection décrit ci-dessus est donc doté d'un système de protection à déclenchement automatique, conçu pour permettre d'administrer le liquide à injecter selon un geste naturel, et dont le déclenchement intervient uniquement une fois la dose intégrale de liquide injectée. De plus, ce dispositif d'injection est conçu pour ne pas pouvoir être réutilisé, sauf bien sûr à dégrader certains éléments de blocage dudit dispositif.

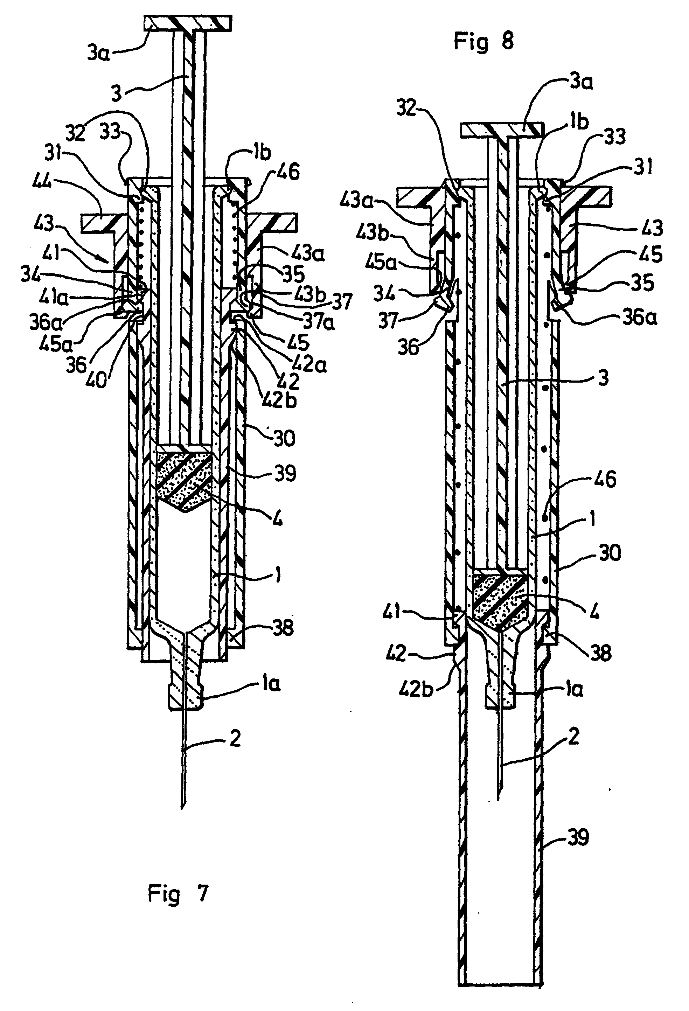

Le dispositif d'injection représenté aux figures 7 et 8 comprend, quant à lui, en premier lieu, outre l'ensemble seringue 1/tige de piston 3/piston 4 décrit plus haut, un corps de seringue 30 constitué d'un fourreau tubulaire d'un diamètre interne supérieur au diamètre externe de la seringue 1, adapté pour loger un étui de protection décrit plus loin dans l'espace annulaire délimité par lesdits corps de seringue et seringue.

Ce fourreau tubulaire 30 de longueur sensiblement inférieure à celle de la seringue 1, comprend, en premier lieu, une nervure interne annulaire postérieure 31 dans laquelle est ménagée une gorge annulaire 32 de blocage de la collerette 1b de ladite seringue.

Ce fourreau tubulaire 30 comprend, également au niveau de son extrémité postérieure, une nervure externe annulaire 33. Ce fourreau tubulaire 30 comprend, en outre, sensiblement au quart de sa longueur à partir de son extrémité postérieure, deux languettes 34, 35 diamétralement opposées, déformables radialement, délimitées chacune par une découpe en forme de ∪ ménagée dans la paroi périphérique dudit fourreau tubulaire.

Chacune de ces languettes 34, 35 comporte, d'une part, un bossage interne 36 possédant une face frontale postérieure 36a inclinée, et d'autre part, un bossage externe 37 possédant une face frontale antérieure 37a inclinée.

Le fourreau tubulaire 30 comporte enfin, au niveau de son extrémité antérieure, une nervure interne annulaire 38 définissant deux faces frontales de butée radiale.

Le dispositif d'injection comprend, par ailleurs, un étui de protection 39 de diamètre interne conjugué du diamètre externe de la seringue, et de diamètre externe conjugué du diamètre interne du fourreau tubulaire 30, au niveau de la nervure 38 de ce dernier.

Cet étui de protection 39 présente, en outre, une longueur adaptée pour que son tronçon d'extrémité postérieur s'étende en regard des languettes 34, 35 du fourreau tubulaire 30, et que son extrémité antérieure s'étende sensiblement dans le prolongement antérieur dudit fourreau tubulaire.

En vue de son blocage en translation dans une position d'éjection où l'aiguille 2 est dégagée, cet étui de protection 39 comporte une gorge externe annulaire 40 apte à loger le bossage interne 36 des languettes 34, 35 et délimitée par deux nervures externes 41, 42 de diamètres externes conjugués du diamètre interne du fourreau tubulaire 30 :
- une nervure postérieure 41 dotée d'une face frontale antérieure 41a inclinée, d'appui contre la face frontale postérieure 36 du bossage 36 des languettes 34, 35,
- une nervure antérieure 42 dotée d'une face frontale postérieure 42a de butée radiale, et d'une face frontale antérieure 42b inclinée.

Le dispositif d'injection comprend, enfin, une bague de verrouillage 43 de diamètre interne conjugué du diamètre externe du fourreau tubulaire 30, et de longueur adaptée pour s'étendre sur environ les trois-quarts de la longueur du tronçon dudit fourreau tubulaire compris entre les extrémités antérieures des languettes 34, 35 et l'extrémité postérieure de ce fourreau tubulaire 30.

Cette bague de verrouillage 43 comprend, en premier lieu, au niveau de son extrémité postérieure, une collerette externe 44 d'appui digital.

Elle présente, en outre, un tronçon postérieur tubulaire 43 de diamètre interne conjugué du diamètre externe du fourreau tubulaire 30,et un tronçon antérieur tubulaire 43b de diamètre sensiblement supérieur à celui du diamètre externe dudit fourreau tubulaire, formant une gorge délimitée par une nervure antérieure interne 45 dotée d'une face frontale postérieure 45a inclinée, d'appui contre la face antérieure inclinée 37a des bossages 37 des languettes 34, 35 du fourreau tubulaire 30.

La fabrication du dispositif d'injection décrit ci-dessus comprend les étapes principales suivantes.

En premier lieu, un ressort 46 est introduit dans le corps de seringue 30, puis l'étui de protection 39 est également introduit dans ce corps de seringue 30 jusqu'à amener la gorge 40 à loger les bossages internes 36 des languettes 34, 35. Dans cette position, le ressort 46 se trouve comprimé entre la face antérieure de la nervure 31 du corps de seringue 30 et la face d'extrémité postérieure de l'étui de protection 39.

La bague de verrouillage 43 est ensuite montée le long du corps de seringue 30 jusqu'à amener la nervure interne 45 à verrouiller le blocage en translation dudit corps de seringue par rapport à l'étui de protection 39, par portée de la face postérieure 36a de la nervure 36 contre la face antérieure 41a de la nervure 41.

La dernière opération consiste à intégrer la seringue pré-remplie 1 en l'introduisant dans l'étui de protection 39 jusqu'à amener la collerette 1b à se bloquer dans la gorge 32 du corps de seringue 30.

Comme le précédent, le dispositif d'injection obtenu, représenté à la figure 7, est ensuite utilisé de façon classique selon un geste naturel consistant à exercer une pression d'appui sur le poussoir 3a et une force de réaction d'appui sur la collerette d'appui digital 44 de la bague de verrouillage 43.

Comme précédemment, ces pression d'appui et force de réaction provoquent en premier lieu le coulissement du piston 4, puis lorsque ce dernier est arrivé enfin de course, conduisent à faire remonter la bague de verrouillage 43 le long du corps de seringue 30, jusqu'à amener cette dernière en butée contre la nervure postérieure 33 dudit corps de seringue.

Les languettes 34, 35 du corps de seringue 30 se trouvent alors déverrouillées, de sorte que l'étui de protection 39 sollicité par le ressort 46 est amené à coulisser le long de la seringue 1 jusqu'à une position avancée où la gorge 40 de ce dernier loge la nervure 38 du corps de seringue 30.

Il est à noter que cette position de blocage en translation de l'étui de protection 39 dans sa position avancée peut être obtenue grâce à l'inclinaison de la face frontale antérieure 42b de la nervure 42 de cet étui de protection 39 qui permet à cette nervure 42 de franchir la nervure 38 du corps de seringue 30.

Par contre, une fois dans sa position avancée, l'étui de protection ne peut pas être rétracté du fait de la butée radiale 42a que forme la face frontale postérieure de la nervure 42.

## Revendications

1. Dispositif d'injection à usage unique comprenant :
- une seringue (1) comportant une aiguille d'injection (2), et délimitant une chambre remplie d'une dose de liquide à injecter, obturée par un piston (4) solidaire d'une des extrémités d'une tige de piston (3) dotée d'un poussoir (3a) au niveau de son extrémité opposée,
- un corps de seringue (5 ; 30) solidaire de la seringue (1),
- un étui de protection (10; 39),
- des moyens (9, 14, 15, 21 ; 34, 35, 38, 40-42) de blocage relatif en translation ménagés sur le corps de seringue (5 ; 30) et l'étui de protection (10 ; 39), pour permettre un déplacement relatif dudit étui de protection et de l'ensemble seringue (1)/corps de seringue (5 ; 30) entre une position d'injection où l'aiguille d'injection (2) est dégagée et une position de protection après usage où ladite aiguille d'injection est logée à l'intérieur de l'étui de protection (10 ; 39), ledit dispositif d'injection,
- des moyens élastiques (27 ; 46) disposés entre le corps de seringue (5 ; 30) et l'étui de protection (10 ; 39) de façon à être comprimés dans la position d'injection de ces derniers, le dispositif d'injection étant **caractérisé par** :
- une bague de verrouillage (23 ; 43) dotée de moyens d'appui digital (24 ; 44) pour l'injection du liquide, et d'organes (25 ; 45) de blocage en translation de ladite bague aptes à la maintenir dans une position où elle verrouille les moyens de blocage en translation (9, 14, 15 ; 34, 35, 40-42) de l'étui de protection (10 ; 39) et de l'ensemble seringue (1)/corps de seringue (5 ; 30), dans la position d'injectién de ces derniers, en l'absence d'effort exercé sur les moyens d'appui digital (24 ; 44) et pour un effort exercé sur ces derniers équivalent à la force de réaction engendrée conduisant au coulisse ment du piston (4), et pour autoriser un déplacement axial de ladite bague vers une position où elle libère lesdits moyens de blocage et autorise le déplacement relatif de l'étui de protection (10 ; 39) et de l'ensemble seringue (1)/corps de seringuè (5 ; 30) vers la position de protection après usage de ces derniers, grâce à l'action des moyens élastiques (27 ; 46), pour un effort exercé sur les moyens d'appui digital (24 ; 44) supérieur à la force de réaction engendrée en vue du coulissement du piston (5), et obtenue une fois ledit piston parvenu en fin de course.

2. Dispositif d'injection selon la revendication 1, **caractérisé en ce que** les moyens élastiques (27) sont disposés entre le corps de seringue (5) et l'étui de protection (10) de façon à engendrer un retrait de l'ensemble seringue (1)/corps de seringue (5) à l'intérieur dudit étui de protection après déplacement axial de la bague de verrouillage (23).

3. Dispositif d'injection selon la revendication 2, **caractérisé en ce que**:
- le corps de seringue comporte une bague tabulaire (5) logée à l'intérieur de l'étui de protection (10), lesdits bague et étui de protection comprenant des épaulements internes (8a, 13) entre lesquels s'étendent les moyens élastiques (27),
- la bague de verrouillage (23) est montée autour de l'étui de protection (10) de façon à pouvoir coulisser axialement par rapport à ce dernier.

4. Dispositif d'injection selon la revendication 3, **caractérisé en ce que** les moyens de blocage relatif en translation (9, 14, 15, 21) de la bague tubulaire (5) et de l'étui de protection (10) sont ménagés de façon que, dans la position de protection après usage, ladite bague tubulaire s'étende au moins partiellement dans le prolongement postérieur dudit étui de protection.

5. Dispositif d'injection selon la revendication 4, **caractérisé en ce que** la bague annulaire (5) présente au moins une surface externe de couleur différente de celle de la bague de verrouillage (23) et de l'étui de protection (10).

6. Dispositif d'injection selon l'une des revendications 3 à 5, **caractérisé en ce que** l'étui de protection (10) comporte au moins une languette (14, 15) déformable radialement portant un élément (16) de blocage relatif en translation dudit étui de protection dans sa position d'injection, la bague de verrouillage (23) étant adaptée pour enserrer chacune desdites languettes, dans sa position de verrouillage; et pour libérer chaque languette (14, 15) de façon à autoriser son expansion radiale, lors de son déplacement axial.

7. Dispositif d'injection selon la revendication 6, **caractérisé en ce que** les moyens de blocage relatif en translation (9, 14, 15) de la bague tabulaire (5) et de l'étui de protection (10), dans la position d'injection de ces derniers, comportent, pour chaque languette (14, 15), un bossage interne (16) ménagé sur cette dernière et une nervure externe (9) ménagée sur la bague tubulaire (5), lesdits bossage et nervure présentant des portées d'appui inclinées (9a, 16) conjuguées.

8. Dispositif d'injection selon la revendication 7, **caractérisé en ce que** les moyens de blocage relatif en translation de la bague tubulaire (5) et de l'étui de protection (10), dans la position de protection après usage de ces derniers, comprennent une nervure interne (21) ménagée vers l'extrémité postérieure dudit étui de protection, présentant une portée d'appui inclinée (21a) conjuguée de celle (9a) de la nervure externe (9) de la bague tubulaire (5).

9. Dispositif d'injection selon la revendication 8, **caractérisé en ce que** l'étui de protection (10) comporte au moins une languette (18, 19) déformable radialement, ménagée à une distance de la nervure postérieure (21) dudit étui de protection adaptée pour assurer le blocage en translation de la nervure externe (9) de la bague tubulaire (5), chacune desdites languettes présentant une position naturelle expansée radialement apte à permettre le montage initial de la bague tubulaire (5), et étant adaptée pour être rétractée radialement de façon à venir en saillie à l'intérieur de l'étui de protection (10), lors du montage de la bague de verrouillage (23).

10. Dispositif d'injection selon l'une des revendications 8 ou 9, **caractérisé en ce que** la bague de verrouillage (23) comporté un tronçon tubulaire postérieur (26) agencé pour ceinturer la nervure interne postérieure (21) de l'étui de protection (10), de façon à fretter cette dernière.

11. Dispositif d'injection selon la revendication 10, **caractérisé en ce que** la bague de verrouillage (23) comporte un épaulement interne (26a) ménagé au niveau de son tronçon tubulaire postérieur (26), l'étui de protection (10) comportant un bossage (22) de butée dudit épaulement après déplacement axial de ladite bague de verrouillage.

12. Dispositif d'injection selon l'une des revendications 3 à 11, **caractérisé en ce que** l'étui de protection (10) comporte un tronçon postérieur (12) de diamètre adapté pour loger la bague tubulaire (5) et le long duquel s'étend la bague de verrouillage (23), et un tronçon antérieur (11) de diamètre conjugué du diamètre externe de la seringue (11), lesdits tronçons postérieur et antérieur étant séparés par un épaulement interne (13) d'appui des moyens élastiques (27).

13. Dispositif d'injection selon l'une des revendications 3 à 12, **caractérisé en ce que** la seringue (1) comporte une collerette postérieure (1b), la bague tubulaire (5) comportant une gorge annulaire interne (7) postérieure de section adaptée pour loger ladite collerette.

14. Dispositif d'injection selon l'une des revendications 3 à 13, **caractérisé en ce que** l'étui de protection (10) comporte une nervure annulaire externe (17), les moyens de verrouillage de la bague de verrouillage (23) consistant en une gorge annulaire interne (25) ménagée dans ladite bague de verrouillage, et de section adaptée pour loger ladite nervure externe.

15. Dispositif d'injection selon la revendication 1, **caractérisé en ce que** les moyens élastiques (46) sont disposés entre le corps de seringue (30) et l'étui de protection (39) de façon à engendrer le coulissement dudit étui de protection le long de la seringue (1), après déplacement axial de la bague de verrouillage (43).

16. Dispositif d'injection selon la revendication 15, **caractérisé en ce que** :
- le corps de seringue comporte un fourreau tubulaire (30) à l'intérieur duquel est logé l'étui de protection (39), les moyens élastiques (46) s'étendant dans ledit fourreau tubulaire dans le prolongement postérieur dudit étui de protection,
- la bague de verrouillage (43) est montée autour du fourreau tubulaire (30) de façon à pouvoir coulisser axialement parrapport à ce dernier.

17. Dispositif d'injection selon la revendication 16, **caractérisé en ce que** le fourreau tubulaire (30) comporte au moins une languette (34, 35) déformable radialement, portant un élément (36) de blocage relatif en translation dudit fourreau dans sa position d'injection, la bague de verrouillage (43) étant adaptée pour enserrer chacune desdites languettes, dans sa position de verrouillage, et pour libérer chaque languette (34, 35) de façon à autoriser son expansion radiale, lors de son déplacement axial.

18. Dispositif d'injection selon la revendication 17, **caractérisé en ce que** les moyens de blocage relatif en translation du fourreau tubulaire (30) et de l'étui de protection (39), dans la position d'injection de ces derniers, comportent, pour chaque languette (34, 35), un bossage interne (36) ménagé sur cette dernière et une nervure externe (41) ménagée sur l'étui de protection (39), lesdits bossage et nervure présentant des portées d'appui inclinées (36a, 41a) conjuguées.

19. Dispositif d'injection selon l'une des revendications 17 ou 18, **caractérisé en ce que** les moyens de blocage relatif en translation du fourreau tubulaire (30) et de l'étui de protection (39), dans la position de protection après usage de ces derniers, comprennent une seconde nervure externe (42) ménagée sur l'étui de protection (39) et délimitant une gorge (40) avec la première nervure externe (41), et une nervure interne (38) ménagée au niveau de l'extrémité antérieure du fourreau tubulaire (30), de forme adaptée pour venir se loger dans la gorge (40).

20. Dispositif d'injection selon l'une des revendications 17 à 18, **caractérisé en ce que** le fourreau tubulaire (30) comporte une nervure externe (37) ménagée sur ledit fourreau au niveau de chaque languette (34, 35), les moyens de verrouillage de la bague de verrouillage (43) consistant en une gorge annulaire interne (43b) délimitée par une nervure antérieure (45) agencée pour se trouver en butée sous la nervure (37) du fourreau tubulaire (30).

21. Dispositif d'injection selon l'une des revendications 16 à 20, **caractérisé en ce que** le fourreau tubulaire (30) comprend une nervure annulaire externe postérieure (33) de butée de la bague de verrouillage (43) après déplacement axial de cette dernière.

22. Dispositif d'injection selon l'une des revendications 16 à 21, **caractérisé en ce que** la seringue (1) comporte une collerette postérieure (1b), le fourreau tubulaire (30) comportant une gorge annulaire interne postérieure (32) de section adaptée pour loger ladite collerette.

## Claims

1. A single-use injection device comprising:
- a syringe (1), comprising an injection needle (2), and delimiting a chamber filled with a dose of fluid to be injected, which is sealed by a plunger (4) which is integral with one of the ends of a plunger rod (3), which is provided with a thruster (3a) at its opposite end;
- a syringe body (5; 30) which is integral with the syringe (1);
- a protective case (10; 39);
- means (9, 14, 15, 21 ; 34, 35, 38, 40-42) for relative locking in translation, provided on the syringe body (5; 30) and the protective case (10; 39), in order to permit relative displacement of the said protective case and of the syringe (1)/syringe body (5; 30) assembly, between a position of injection, in which the injection needle (2) is released, and a position of protection after use, in which the said injection needle is accommodated inside the protective case (10; 39);
- resilient means (27; 46), which are disposed between the syringe body (5; 30) and the protective case (10; 39), such as to be compressed in the position of injection of the latter, the injection device being **characterized by**:
- a locking ring (23; 43), which is provided with digital support means (24; 44) for injection of the fluid, and units (25; 45) for locking in translation of the said ring, which can maintain the ring in a position in which it locks the means for locking in translation (9, 14, 15; 34, 35, 40-42) of the protective case (10; 39) and of the syringe (1)/syringe body (5; 30) assembly, in the position of injection of the latter, in the absence of force exerted on the digital support means (24; 44), and for a force exerted on the latter, which is equivalent to the reaction force generated, thus leading to sliding of the plunger (4), and in order to permit axial displacement of the said ring towards a position in which it releases the said locking means, and permits relative displacement of the protective case (10; 39) and of the syringe (1)/syringe body (5; 30) assembly, towards the position of protection, after use of the latter, by means of the action of the resilient means (27; 46), for a force exerted on the digital support means (24; 44), which is greater than the reaction force which is generated for the purpose of sliding of the plunger (5), and is obtained when the said plunger has reached its end of travel.

2. Injection device according to claim 1, **characterized in that** the resilient means (27) are disposed between the syringe body (5) and the protective case (10), such as to generate withdrawal of the syringe (1)/syringe body (5) assembly inside the said protective case, after axial displacement of the locking ring (23).

3. Injection device according to claim 2, **characterized in that**:
- the syringe body comprises a tubular ring (5), which is accommodated inside the protective case (10), the said ring and protective case comprising inner shoulders (8a, 13), between which the resilient means (27) extend; and
- the locking ring (23) is fitted around the protective case (10), such as to be able to slide axially relative to the latter.

4. Injection device according to claim 3, **characterized in that** the means (9, 14, 15, 21) for relative locking in translation of the tubular ring (5) and of the protective case (10) are disposed such that, in the position of protection after use, the tubular ring extends at least partially in the rear extension of the said protective case.

5. Injection device according to claim 4, **characterized in that** the annular ring (5) has at least one outer surface with a colour different from that of the locking ring (23) and of the protective case (10).

6. Injection device according to any one of claims 3 to 5, **characterized in that** the protective case (10) comprises at least one tab (14, 15) which can be deformed radially, which supports an element (16) for relative locking in translation of the said protective case in its position of injection, the locking ring (23) being designed to clamp each of the said tabs in its locking position, and to release each tab (14, 15), such as to permit radial expansion of the latter, during its axial displacement.

7. Injection device according to claim 6, **characterized in that** the means (9, 14, 15) for relative locking in translation of the tubular ring (5) and of the protective case (10), in the position of injection of the latter, comprise for each tab (14, 15) an inner boss (16), which is provided on the latter, and an outer rib (9), which is provided on the tubular ring (5), the said boss and rib having conjugated inclined support surfaces (9a, 16).

8. Injection device according to claim 7, **characterized in that** the means for relative locking in translation of the tubular ring (5) and of the protective case (10), in the position of protection after use of the latter, comprise an inner rib (21), which is provided towards the rear end of the said protective case, and has an inclined support surface (21a) which is conjugated with that (9a) of the outer rib (9) of the tubular ring (5).

9. Injection device according to claim 8, **characterized in that** the protective case (10) comprises at least one tab (18, 19) which can be deformed radially, which is provided spaced from the rear rib (21) of the said protective case, and is designed to assure the locking in translation of the outer rib (9) of the tubular ring (5), each of the said tabs having a natural, radially expanded position, which can permit initial fitting of the tubular ring (5), and is designed to be retracted radially, such as to project inside the protective case (10), during fitting of the locking ring (23).

10. Injection device according to claim 8 or claim 9, **characterized in that** the locking ring (23) comprises a rear tubular section (26), which is designed to surround the rear inner rib (21) of the protective case (10), such as to shrink the latter on.

11. Injection device according to claim 10, **characterized in that** the locking ring (23) comprises an inner shoulder (26a), which is provided at the level of its rear tubular section (26), the protective case (10) comprising a boss (22) to stop the said shoulder, after the said locking ring has been displaced axially.

12. Injection device according to any one of claims 3 to 11, **characterized in that** the protective case (10) comprises a rear section (12) with a diameter which is designed to accommodate the tubular ring (5), and along which the locking ring (23) extends, and a front section (11), with a diameter which is conjugated with the outer diameter of the syringe (11), the said rear and front sections being separated by an inner shoulder (13) for support of the resilient means (27).

13. Injection device according to any one of claims 3 to 12, **characterized in that** the syringe (1) comprises a rear collar (1b) the tubular ring (5) comprising a rear inner annular groove (7), with a cross-section which is designed to accommodate the said collar.

14. Injection device according to any one of claims 3 to 13, **characterized in that** the protective case (10) comprises an outer annular rib (17), the means for locking the locking ring (23) consisting of an inner annular groove (25) which is provided in the said locking ring, and has a cross-section which is designed to accommodate the said outer rib.

15. Injection device according to claim 1, **characterized in that** the resilient means (46) are disposed between the syringe body (30) and the protective case (39), such as to give rise to sliding of the said protective case along the syringe (1), after axial displacement of the locking ring (43).

16. Injection device according to claim 15, **characterized in that**:
- the syringe body comprises a tubular sheath (30), inside which the protective case (39) is accommodated, the resilient means (46) extending in the said tubular sheath, in the rear extension of the said protective case;
- the locking ring (43) is fitted around the tubular sheath (30), such as to be able to slide axially relative to the latter.

17. Injection device according to claim 16, **characterized in that** the tubular sheath (30) comprises at least one tab (34, 35) which can be deformed radially, which supports an element (36) for relative locking in translation of the said sheath in its position of injection, the locking ring (43) being designed to clamp each of the said tabs in its locking position, and to release each tab (34, 35), such as to permit radial expansion of the latter, during its axial displacement.

18. Injection device according to claim 17, **characterized in that** the means for relative locking in translation of the tubular sheath (30) and of the protective case (39), in the position of injection of the latter, comprise for each tab (34, 35) an inner boss (36) which is provided on the latter, and an outer rib (41), which is provided on the protective case (39), the said boss and rib having conjugated inclined support surfaces (36a, 41a).

19. Injection device according to claim 17 or claim 18,
**characterized in that** the means for relative locking in translation of the tubular sheath (30) and of the protective case (39), in the position of protection after use of the latter, comprise a second outer rib (42), which is provided on the protective case (39), and delimits a groove (40) together with the first outer rib (41), and an inner rib (38), which is provided at the level of the front end of the tubular sheath (30), and has a shape which is designed to be accommodated in the groove (40).

20. Injection device according to claim 17 or claim 18, **characterized in that** the tubular sheath (30) comprises an outer rib (37), which is provided on the said sheath at the level of each tab (34, 35), the means for locking the locking ring (43) consisting of an inner annular groove (43b), which is delimited by a front rib (45), which is designed to abut beneath the rib (37) of the tubular sheath (30).

21. Injection device according to any one of claims 16 to 20, **characterized in that** the tubular sheath (30) comprises a rear outer annular rib (33), to stop the locking ring (43) after axial displacement of the latter.

22. Injection device according to any one of claims 16 to 21, **characterized in that** the syringe (1) comprises a rear collar (1b), the tubular sheath (30) comprising a rear inner annular groove (32), with a cross-section which is designed to accommodate the said collar.

## Patentansprüche

1. Einweginjektionsvorrichtung, die Folgendes umfasst:
- eine Spritze (1) die eine Injektionsnadel (2) umfasst und eine Kammer begrenzt, die mit einer zu injizierenden Flüssigkeitsdosis gefüllt ist und von einem Kolben (4) verschlossen wird, der einstückig mit einem der Enden einer Kolbenstange (3) ausgebildet ist, die in der Höhe ihres gegenüberliegenden Endes mit einem Vordrücker (3a) versehen ist,
- einen mit der Spritze (1) einstückigen Spritzenkörper (5; 30),
- eine Schutzhülse (10; 39),
- Mittel (9, 14, 15, 21; 34, 35, 38, 40-42) zum Sperren einer relativen Translationsbewegung, die an dem Spritzenkörper (5; 30) und der Schutzhülse (10; 39) vorgesehen sind, um eine relative Verschiebung der genannten Schutzhülse und der Baugruppe aus Spritze (1) und Spritzenkörper (5; 30) zwischen einer Injektionsposition, in der die Injektionsnadel (2) frei ist, und einer Nachgebrauchs-Schutzposition zulässt, in der die genannte Injektionsnadel im Inneren der Schutzhülse (10; 39) aufgenommen wird,
- elastische Mittel (27; 46), die so zwischen dem Spritzenkörper (5; 30) und der Schutzhülse (10; 39) angeordnet sind, dass sie in der Injektionsposition der Letzteren zusammengedrückt werden, wobei die Injektionsvorrichtung **gekennzeichnet ist durch**:
- einen Sperrring (23; 43), der mit Fingeranlagemitteln (24; 44) zum Injizieren der Flüssigkeit und mit Organen (25; 45) zum Sperren einer Translationsbewegung des genannten Rings versehen ist, um diesen in einer Position zu halten, in der er die Translationsbewegungssperrmittel (9, 14, 15; 34, 35, 40-42) der Schutzhülse (10; 39) und der Baugruppe aus Spritze (1) und Spritzenkörper (5; 30) in der Injektionsposition des Letzteren in Abwesenheit einer Kraft auf die Fingeranlagemittel (24; 44) und für eine Kraft auf die Letzteren sperrt, die äquivalent zu der erzeugten Gegenkraft ist, die zur Verschiebung des Kolbens (4) führt, und um eine axiale Verschiebung des genannten Rings in eine Position zuzulassen, in der er die genannten Sperrmittel freisetzt und eine relative Verschiebung der Schutzhülse (10; 39) und der Baugruppe aus Spritze (1) und Spritzenkörper (5; 30) in Richtung auf die Nachgebrauchs-Schutzposition der Letzteren dank der Wirkung der elastischen Mittel (27; 46) für eine Kraft auf die Fingeranlagemittel (24; 44) zulässt, die größer ist als die erzeugte Gegenkraft zum Verschieben des Kolbens (5), und die erzielt wird, wenn der genannte Kolben am Ende des Weges angelangt ist.

2. Injektionsvorrichtung nach Anspruch 1, **dadurch gekennzeichnet, dass** die elastischen Mittel (27) so zwischen Spritzenkörper (5) und Schutzhülse (10) angeordnet sind, dass sie ein Zurückziehen der Baugruppe aus Spritze (1) und Spritzenkörper (5) ins Innere der genannten Schutzhülse nach der axialen Verschiebung des Sperrrings (23) bewirken.

3. Injektionsvorrichtung nach Anspruch 2, **dadurch gekennzeichnet, dass**:
- der Spritzenkörper einen röhrenförmigen Ring (5) aufweist, der im Inneren der Schutzhülse (10) aufgenommen wird, wobei der genannte Ring und die genannte Schutzhülse Innenansätze (8a, 13) aufweisen, zwischen denen die elastischen Mittel (27) verlaufen,
- der Sperrring (23) so um die Schutzhülse (10) montiert ist, dass er in Bezug auf die Letztere axial gleiten kann.

4. Injektionsvorrichtung nach Anspruch 3, **dadurch gekennzeichnet, dass** die relativen Translationsbewegungssperrmittel (9, 14, 15, 21) des röhrenförmigen Rings (5) und der Schutzhülse (10) so angeordnet sind, dass sich der genannte röhrenförmige Ring in der Nachgebrauchs-Schutzposition wenigstens teilweise in der hinteren Verlängerung der genannten Schutzhülse befindet.

5. Injektionsvorrichtung nach Anspruch 4, **dadurch gekennzeichnet, dass** der ringförmige Ring (5) wenigstens eine Außenfläche mit einer Farbe aufweist, die sich von der des Sperrrings (23) und der Schutzhülse (10) unterscheidet.

6. Injektionsvorrichtung nach einem der Ansprüche 3 bis 5, **dadurch gekennzeichnet, dass** die Schutzhülse (10) wenigstens eine radial verformbare Zunge (14, 15) aufweist, die ein Element (16) zum relativen Sperren einer Translationsbewegung der genannten Schutzhülse in ihrer Injektionsposition trägt, wobei der Sperrring (23) so gestaltet ist, dass er jede der genannten Zungen in ihrer Sperrposition verriegelt, und um jede Zunge (14, 15) so freizusetzen, dass sie während ihrer axialen Verschiebung radial expandieren kann.

7. Injektionsvorrichtung nach Anspruch 6, **dadurch gekennzeichnet, dass** die Mittel zum relativen Sperren einer Translationsbewegung (9, 14, 15) des röhrenförmigen Rings (5) und der Schutzhülse (10) in der Injektionsposition der Letzteren für jede Zunge (14, 15) einen Innenvorsprung (16), der an der Letzteren vorgesehen ist, und eine am röhrenförmigen Ring (5) vorgesehene Außenrippe (9) umfasst, wobei der genannte Vorsprung und die genannte Rippe zusammenpassende geneigte Widerlager (9a, 16) bilden.

8. Injektionsvorrichtung nach Anspruch 7, **dadurch gekennzeichnet, dass** die relativen Translationssperrmittel des röhrenförmigen Rings (5) und der Schutzhülse (10) in der Nachgebrauchs-Schutzposition der Letzteren eine Innenrippe (21) umfassen, die in Richtung auf das hintere Ende der genannten Schutzhülse vorgesehen ist und ein geneigtes Widerlager (21a) bildet, das zu dem (9a) der Außenrippe (9) des röhrenförmigen Rings (5) passt.

9. Injektionsvorrichtung nach Anspruch 8, **dadurch gekennzeichnet, dass** die Schutzhülse (10) wenigstens eine radial verformbare Zunge (18, 19) aufweist, die in einem Abstand von der hinteren Rippe (21) der genannten Schutzhülse vorgesehen und so gestaltet ist, dass sie eine Translationssperrung der Außenrippe (9) des röhrenförmigen Rings (5) gewährleistet, wobei jede der genannten Zungen eine natürliche radiale expandierte Position aufweist, die eine anfängliche. Montage des röhrenförmigen Rings (5) zulässt, und die so gestaltet ist, dass sie radial so zurückgezogen werden kann, dass sie während der Montage des Sperrrings (23) ins Innere der Schutzhülse (10) auskragt.

10. Injektionsvorrichtung nach Anspruch 8 oder 9, **dadurch gekennzeichnet, dass** der Sperrring (23) ein hinteres röhrenförmiges Endstück (26) aufweist, das die Aufgabe hat, die hintere Innenrippe (21) der Schutzhülse (10) so zu umgeben, dass es die Letztere bandagiert.

11. Injektionsvorrichtung nach Anspruch 10, **dadurch gekennzeichnet, dass** der Sperrring (23) einen Innenansatz (26a) in der Höhe seines röhrenförmigen Endstücks (26) aufweist, wobei die Schutzhülse (10) einen Widerlagervorsprung (22) für den genannten Ansatz nach der axialen Verschiebung des genannten Sperrrings aufweist.

12. Injektionsvorrichtung nach einem der Ansprüche 3. bis 11, **dadurch gekennzeichnet, dass** die Schutzhülse (10) ein hinteres Endstück (12) mit einem Durchmesser, der geeignet ist, den röhrenförmigen Ring (5) aufzunehmen, und entlang dessen der Sperrring (23) verläuft, und ein vorderes Endstück (11) mit einem Durchmesser aufweist, der zum Außendurchmesser der Spritze (11) passt, wobei das genannte vordere und das genannte hintere Endstück durch einen Innenanlageansatz (13) für die elastischen Mittel (27) getrennt sind.

13. Injektionsvorrichtung nach einem der Ansprüche 3 bis 12, **dadurch gekennzeichnet, dass** die Spritze (1) einen hinteren Kragen (1b) aufweist, wobei der röhrenförmige Ring (5) eine hintere ringförmige Innenauskehlung (7) mit einem Querschnitt aufweist, der den genannten Kragen aufnimmt.

14. Injektionsvorrichtung nach einem der Ansprüche 3 bis 13, **dadurch gekennzeichnet, dass** die Schutzhülse (10) eine ringförmige Außenrippe (17) aufweist, wobei die Sperrmittel des Sperrrings (23) aus einer ringförmigen Innenauskehlung (25) bestehen, die in dem genannten Sperrring vorgesehen ist und einen Querschnitt hat, der so gestaltet ist, dass er die genannte Außenrippe aufnimmt.

15. Injektionsvorrichtung nach Anspruch 1, **dadurch gekennzeichnet, dass** die elastischen Mittel (46) zwischen dem Spritzenkörper (30) und der Schutzhülse (39) so angeordnet sind, dass sie das Gleiten der genannten Schutzhülse entlang der Spritze (1) nach der axialen Verschiebung des Sperrrings (43) bewirken.

16. Injektionsvorrichtung nach Anspruch 15, **dadurch gekennzeichnet, dass**:
- der Spritzenkörper ein röhrenförmiges Futter (30) aufweist, in dessen Inneren die Schutzhülse (39) aufgenommen wird, wobei die elastischen Mittel (46) in dem genannten röhrenförmigen Futter in der hinteren Verlängerung der genannten Schutzhülse verlaufen,
- der Sperrring (43) so um das röhrenförmige Futter (30) montiert ist, dass er axial in Bezug auf Letzteres gleiten kann.

17. Injektionsvorrichtung nach Anspruch 16, **dadurch gekennzeichnet, dass** das röhrenförmige Futter (30) wenigstens eine radial verformbare Zunge (34, 35) aufweist, die ein relatives Translationssperrelement (36) des genannten Futters in der Injektionsposition trägt, wobei der Sperrring (43) so gestaltet ist, dass er in seiner Sperrposition jede der genannten Zungen verriegelt, und bei seiner axialen Verschiebung jede Zunge (34, 35) so freigibt, dass sie radial expandieren kann.

18. Injektionsvorrichtung nach Anspruch 17, **dadurch gekennzeichnet, dass** die relativen Translationssperrmittel des röhrenförmigen Futters (30) und der Schutzhülse (39) in der Injektionsposition der Letzteren für jede Zunge (34, 35) einen Innenvorsprung (36), der an Letzterem vorgesehen ist, und eine Außenrippe (41) aufweist, die an der Schutzhülse (39) vorgesehen ist, wobei der genannte Vorsprung und die genannte Rippe zusammenpassende geneigte Widerlager (36a, 41a) bilden.

19. Injektionsvorrichtung nach Anspruch 17 oder 18, **dadurch gekennzeichnet, dass** die relativen Translationssperrmittel des röhrenförmigen Futters (30) und der Schutzhülse (39) in der Nachgebrauchs-Schutzposition der Letzteren eine zweite Außenrippe (42), die an der Schutzhülse (39) vorgesehen ist und eine Auskehlung (40) mit der ersten Außenrippe (41) begrenzt, und eine Innenrippe (38) aufweist, die in der Höhe des vorderen Endes des röhrenförmigen Futters (30) in einer Form vorgesehen ist, die so gestaltet ist, dass sie in der Auskehlung (40) aufgenommen wird.

20. Injektionsvorrichtung nach einem der Ansprüche 17 bis 18, **dadurch gekennzeichnet, dass** das röhrenförmige Futter (30) eine Außenrippe (37) aufweist, die an dem genannten Futter in der Höhe jeder Zunge (34, 35) vorgesehen ist, wobei die Sperrmittel des Sperrrings (43) aus einer ringförmigen Innenauskehlung (43b) besteht, die durch eine vordere Rippe (45) begrenzt wird, deren Zweck es ist, unter der Rippe (37) des röhrenförmigen Futters (30) in Anlage zu kommen.

21. Injektionsvorrichtung nach einem der Ansprüche 16 bis 20, **dadurch gekennzeichnet, dass** das röhrenförmige Futter (30) eine hintere ringförmige Außenrippe (33) für die Anlage des Sperrrings (43) nach der axialen Verschiebung des Letzteren umfasst.

22. Injektionsvorrichtung nach einem der Ansprüche 16 bis 21, **dadurch gekennzeichnet, dass** die Spritze (1) einen hinteren Kragen (1b) umfasst, wobei das röhrenförmige Futter (30) eine hintere ringförmige Innenauskehlung (32) mit einem Querschnitt aufweist, der so gestaltet ist, dass er den genannten Kragen aufnimmt.
